# EUROPEAN PATENT APPLICATION

(11) **EP 4 342 318 A1**
(43) Date of publication of application: **27.03.2024**
(21) Application number: 22803615.8
(22) Date of filing: 17.03.2022
(51) Int. Cl.: A24F 47/00, G16H 50/30, A24F 40/50

(54) **CARTRIDGE, AND AEROSOL GENERATING APPARATUS AND SYSTEM**

(30) Priority: 21.05.2021 CN 202110563107
(71) Applicant: Changzhou Patent Electronic Technology Co., Ltd, Changzhou, Jiangsu 213125 (CN)
(72) Inventor: QIU, Weihua, hangzhou, Jiangsu 213125 (CN)
(74) Representative: Zaboliene, Reda
(86) International application number: PCT/CN2022/081300
(87) International publication number: WO 2022/242286

(57) **Abstract**

A cartridge (1), and an aerosol generating apparatus and system, which solve the problem that the number of times a cartridge (1) has been lit as calculated during operation by an aerosol generating apparatus is inaccurate. The aerosol generating apparatus controls, according the number of times a power supply apparatus (2) has performed lighting, whether an atomization apparatus is turned on or off. The cartridge (1) comprises an encryption chip (11), and the encryption chip (11) comprises: a signal transceiver (111), which is used to receive a lighting signal that is sent by the power supply apparatus (2) and that cooperates with the cartridge (1) in which the encryption chip (11) is located, and send an update signal to a processor (113); a memory (112), which is used to store the number of remaining inhalations as well as a preset maximum number of inhalations, the initial value of the remaining number of inhalations being the maximum number of inhalations; and a processor (113), which is used to receive the update signal sent by the signal transceiver (111), and update the number of remaining inhalations in the memory (112) according to a preset first rule, so that the power supply apparatus (2) cooperating with the cartridge (1) reads, by means of an electrical connection, the number of remaining inhalations stored therein, and then controls an atomization apparatus to stop operation.

## Description

### TECHNICAL FIELD

The disclosure belongs to the technical field of aerosol generating apparatuses, and specifically relates to a cartridge, an aerosol generating apparatus and a system.

### BACKGROUND

The aerosol generating apparatus is a relatively common electronic product that simulates cigarettes and is mainly configured to quit smoking and replace cigarettes. The structure of the aerosol generating apparatus mainly includes a power supply device and a cartridge. The power supply device is provided with a slot for loading cartridges. When the cartridge is loaded into the power supply device and the power supply device collects the cigarette lighting trigger information, the battery component on the power supply device supplies power to the atomizing device of the cartridge; the heating wire in the atomizing device generates heat, heating and atomizing the smoke liquid from the liquid storage chamber in the cartridge, forming smoke for the user to inhale.

Aerosol generating apparatuses currently on the market, in the early stages of smoking, the power and e-liquid are sufficient, and the smoke tastes pure; in the later stages of smoking, due to insufficient battery or reduced e-liquid, the amount of smoke decreases. Therefore, when using this aerosol generating apparatus, the user's smoking liquid tastes inconsistent between the first and second stages of use.

### SUMMARY

The present disclosure provides a cartridge and an aerosol generating apparatus and system, which are configured to solve the problems in the existing technology: when the aerosol generating apparatus consisting of a detachable cartridge and a power supply device is in use, the problem of inconsistent taste caused by the reduction in the amount of e-liquid when users smoke.

In order to solve the above technical problems, some aspects of the present disclosure provide a cartridge, the cartridge cooperates with the power supply device of the aerosol generating apparatus and is electrically connected to the power supply device, the cartridge includes an encryption chip, the encryption chip is configured to limit the number of cigarette lighting times of the power supply device cooperated with the cartridge.

Further, the encryption chip includes a signal transceiver, a memory and a processor;
the signal transceiver is configured to receive a cigarette lighting signal sent by the power supply device that cooperates with the cartridge where the encryption chip is located, and send an update signal to the processor;
the memory is configured to store a remaining inhalation number and a preset maximum inhalation number, and the initial value of the remaining inhalation number is the maximum inhalation number;
the processor is configured to receive an update signal sent by the signal transceiver, and update the remaining inhalation number in the memory according to a preset first rule.

Further, the processor updating the remaining inhalation number in the memory according to a preset first rule, including:
the processor receiving the update signal sent by the signal transceiver, and subtracting the unit inhalation from the remaining inhalation number in the memory, using the calculated value as a new remaining inhalation number and sending the new remaining inhalation number to the memory for update and storage.

Further, the encryption chip includes a signal transceiver, a memory and a processor;
the signal transceiver is configured to receive the cigarette lighting signal sent by the power supply device that cooperates with the cartridge where the encryption chip is located, and send an update signal to the processor;
the memory is configured to store the taken inhalation number and the preset maximum inhalation number;
the processor is configured to receive the update signal sent by the signal transceiver and update the inhalation number in the memory according to a preset first rule.

Further, the maximum inhalation number is pre-recorded in the memory.

The present disclosure further provides an aerosol generating apparatus, including the above-mentioned cartridge, and a power supply device cooperated with the cartridge; the power supply device includes:
a collection unit, configured to collect a cigarette lighting trigger information and send the cigarette lighting trigger information to a control unit;
a reading unit, configured to read the remaining inhalation number stored in the encryption chip, and send the collected remaining inhalation number to the control unit,
the control unit, configured to send a control instruction to a communication unit and send the cigarette lighting signal to the cigarette lighting unit according to the cigarette lighting trigger information, and, detect the value of the remaining inhalation number, and send a cigarette lighting prohibition signal to the cigarette lighting unit when the remaining inhalation number is 0,;
the communication unit, configured to send the cigarette lighting signal to the cartridge according to the control instruction;
the cigarette lighting unit, configured to receive the cigarette lighting signal from the control unit to control the operation of the atomizing device, and further configured to receive the cigarette lighting prohibition signal from the control unit to prohibit the operation of the atomizing device.

The present disclosure further provides an aerosol generating apparatus, including the cartridge according to claim 4, and a power supply device cooperated with the cartridge, and the power supply device includes:
a reading unit, configured to read the inhalation number stored in the encryption chip, and send the collected inhalation number to a control unit;
the control unit, configured to send a control instruction to a communication unit and send the cigarette lighting signal to the cigarette lighting unit according to the cigarette lighting trigger information, and subtract the taken inhalation number from the preset maximum inhalation number to obtain the remaining inhalation number, and send a cigarette lighting prohibition signal to the cigarette lighting unit when the remaining inhalation number is 0;
the communication unit, configured to send the cigarette lighting signal to the cartridge according to the control instruction;
the cigarette lighting unit, configured to receive the cigarette lighting signal from the control unit to control the operation of the atomizing device, and receive a cigarette lighting prohibition signal from the control unit to prohibit the operation of the atomizing device.

Further, the power supply device further includes an alarm unit; the control unit is further configured to control the activation of the alarm unit according to the cigarette lighting prohibition signal.

Further, the encryption chip of the cartridge further stores cartridge information; the power supply device includes:
a reading unit, configured to read a cartridge information in the encryption chip of the cartridge that is in contact with the power supply device where the reading unit is located; a storage unit, configured to store cartridge information of standard cartridges that match the power supply device; a control unit, configured to control the normal operation of the power supply device according to whether the cartridge information sent by the reading unit is consistent with the cartridge information stored in the storage unit.

Further, the control unit is further configured to control the activation of the alarm device of the power supply device according to the stop command signal.

The present disclosure further provides an aerosol generating apparatus system, including the aerosol generating apparatus according to any one of the above claims, and further includes a terminal and/or server,
wherein the control unit of the power supply device is further configured to calculate the change parameter x related to the inhalation number p in the N+1th period of time and the inhalation number m in the Nth period of time, further configured to send the change parameter x to the communication unit, wherein, the Nth period of time and the N+1th period of time are equal in length, N and m are natural numbers, x is 0 or a positive rational number;
the communication unit of the power supply device is further configured to send the change parameter x to the terminal and/or server;
the terminal and/or server includes:
a signal transceiver device, configured to receive the changing parameter x and send it to the control device;
a storage device, configured to pre-store several threshold ranges of the change parameter x, wherein each threshold range is provided with corresponding display content;
the control device, configured to calculate that the change parameter x falls within the q-th preset threshold range, and display the content corresponding to the q-th threshold range to the user, where q is a natural number.

Further, the change parameter x=(m-p)/p, or x=m/p.

The beneficial effects of the present disclosure are: the cartridge provided by this application can itself calculate the number of cigarette lighting times in the power supply device, and limit whether the power supply device works normally based on the number of cigarette lighting times. Since the function of this solution to calculate the number of cigarette lighting times of the power supply device is implemented by the cartridge, if the user replaces the cartridge, the number of cigarette lighting times of the power supply device will be recalculated by another cartridge. Therefore, the number of cigarette lighting times calculated in this plan is calculated from the time when the cartridge is loaded into the power supply device. Compared with the prior art that the number of cigarette lighting starts from the power supply device putting into use, the number of cigarette lighting times calculated in this application is more accurate, more precise restrictions have been placed on the remaining amount of e-liquid in the cartridge. It further solves the problem that the calculated number of cigarette lighting times does not correspond to the cartridges.

### BRIEF DESCRIPTION OF THE DRAWINGS

FIG. 1 is a schematic structural diagram of a cartridge provided by the first embodiment of the present disclosure;
FIG. 2 is a unit schematic diagram of an aerosol generating apparatus provided by the first embodiment of the present disclosure;
FIG. 3 is a flow chart of a control method of an aerosol generation system provided by a fifth embodiment of the present disclosure;
FIG. 4 is a schematic unit diagram of a control system of an aerosol generation system provided by the fifth embodiment of the present disclosure.

In the attached picture: Cartridge 1, Encryption Chip 11, Signal Transceiver 111, Memory 112, Processor 113, Power Supply Device 2, Collection Unit 21, Reading Unit 22, Storage Unit 23, Control Unit 24, Cigarette lighting Unit 25, Alarm Unit 26, Communication Unit 27, Signal Transceiver 31, Storage Device 32, Control Device 33.

### DETAILED DESCRIPTION OF PREFERRED EMBODIMENTSDETAILED DESCRIPTION OF PREFERRED EMBODIMENTS

The following is a further detailed description through specific implementation methods:
In order to make the objectives, technical solutions and advantages of the embodiments of the present disclosure clearer, each implementation mode of the present disclosure will be described in detail below with reference to the accompanying drawings. However, those of ordinary skill in the art will understand that in various embodiments of the present disclosure, many technical details are provided to enable readers to better understand the present application. Even without these technical details and various changes and modifications based on the following embodiments, the technical solution claimed in this application can further be implemented.

In the present disclosure, a server is a computer or device that provides and manages network resources on the network. A terminal may refer to various types of devices, including (but not limited to) wireless telephones, wireless communications personal computers, multimedia wireless devices, external or internal modems, and the like. The terminal may be any data device that communicates with the aerosol generating apparatus and/or server via a wireless channel and/or via a wired channel (eg, optical or coaxial cable). A terminal may have various names, such as mobile station, mobile device, mobile unit, mobile phone, remote station, remote terminal, remote unit, handheld device, etc. Different terminals can be integrated into one system, terminals can be mobile or fixed, and can be dispersed throughout a communications network.

### A first embodiment

This embodiment provides a cartridge 1, the cartridge 1 cooperates with the power supply device 2 of an aerosol generating apparatus and establishes an electrical connection with the power supply device 2, the cartridge 1 includes an encryption chip 11, the encryption chip 11 is configured to limit the number of cigarette lighting times of the power supply device 2 cooperated with the cartridge 1. Among them, the mutual cooperation between the cartridge 1 and the power supply device 2 of the aerosol generating apparatus means that they form a physical connection, optional, detachable connection.

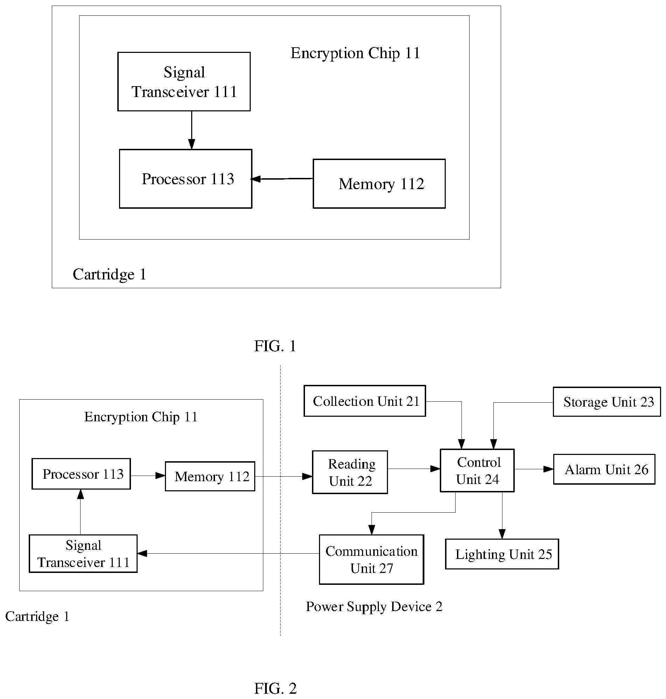

As shown in FIG. 1, the encryption chip 11 includes a signal transceiver 111, a memory 112 and a processor 113.

In one embodiment, the signal transceiver 111 is configured to receive the cigarette lighting signal sent by the power supply device 2 that cooperated with the cartridge 1 where the encryption chip 11 is located, and send an update signal to the processor 113; the memory 112 is configured to store the remaining inhalation number and the preset maximum inhalation number, wherein the initial value of the remaining inhalation number is set to the maximum inhalation number; the processor 113 is configured to receive the update signal sent by the signal transceiver 111 and update the remaining inhalation number in the memory 112 according to the preset first rule, so that the power supply device that cooperates with the cartridge can read the remaining inhalation number stored therein through an electrical connection, and then control atomizing device stops working.

In this embodiment, the preset first rule is: the initial value of the remaining inhalation number is set to the maximum inhalation number, each time the update signal is received, the processor 113 subtracts the unit inhalation number from the remaining inhalation number and takes the difference as a new remaining inhalation number, and updates the remaining inhalation number in the memory 112.

In practical applications, the cartridge 1 provided by this application can calculate the number of cigarette lighting times in the power supply device 2 by itself, and limit whether the power supply device 2 works normally based on the number of cigarette lighting times. Since the function of calculating the number of cigarette lighting times of the power supply device 2 in this application is implemented by the cartridge 1, if the user replaces cartridge 1, the number of cigarette lighting times of power supply device 2 will be recalculated by another cartridge 1. Therefore, the number of cigarette lighting times calculated in this application is calculated from the time when the cartridge 1 is loaded into the power supply device 2, compared with the existing technology, the remaining amount of smoke liquid in the cartridge 1 is more accurately limited, and there is no problem that the calculated number of cigarette lighting times does not correspond to the cartridge 1.

The following is a detailed description of the implementation details of the cartridge 1 of this embodiment. The following content is only provided for the convenience of understanding. The cartridge 1 in this embodiment is applied to an aerosol generating apparatus.

In some embodiments, the aerosol generating apparatus includes a power supply device 2 and a cartridge 1, as shown in FIG.2, the power supply device 2 loaded in the cartridge 1 may be the same model as the cartridge 1, or it may be a different model. After the cartridge 1 is loaded into the power supply device 2, the power supply device 2 will detect whether the cartridge 1 is the same model as the power supply device 2. Only when the detect results is that they are the same model, the power supply device 2 is allowed to read the remaining inhalation number of the cartridge 1. Furthermore, when the remaining inhalation number is 0, the cigarette lighting unit 25 is controlled to stop working and the alarm device is activated. Similarly, the power supply device 2 will only allow the power supply device 2 to send cigarette lighting information to the cartridge 1 when it determines that the model of the cartridge 1 is consistent with its own model.

Among them, there are many methods to determine whether the model of the cartridge 1 is consistent with that of the power supply device 2, for example: the shell of cartridge 1 is engraved with its own model number, the housing of power supply device 2 is further engraved/sprayed with its own model, and then the models of cartridge 1 and power supply device 2 are manually identified.

For another example: the memory 112 of the encryption chip 11 of the cartridge 1 has cartridge information pre-stored; the power supply device 2 is provided with a collection unit 21, a reading unit 22, a storage unit 23, a control unit 24, an cigarette lighting unit 25 and an alarm unit 26.

The cartridge 1 is loaded into power supply device 2, the encryption chip 11 of the cartridge 1 is in direct contact with the reading unit 22 to establish information communication, and that is, the reading unit 22 can read the content in the encryption chip 11. Since the encryption chip 11 stores cartridge information, the reading unit 22 can read the content (including cartridge information) in the encryption chip 11 loaded in the cartridge 1 of the power supply device 2.

The storage unit 23 of the power supply device 2 stores cartridge information of standard cartridges that match the power supply device 2 where it is located.

The control unit 24 determines whether the cartridge information sent by the reading unit 22 is consistent with the cartridge information stored in the storage unit 23: if not, the cigarette lighting unit 25 is configured to control the atomizing device to stop working, and the alarm unit 26 is controlled to start and alarm; if so, the entire power supply device 2 operates normally.

### When the cartridge matches the power supply device, the power supply device works normally

When the user uses it, he presses the button on the power supply device 2 to generate cigarette lighting trigger information;
the collection unit 21 collects the cigarette lighting trigger information and sends the cigarette lighting trigger information to the control unit 24;
The reading unit 22 reads the remaining inhalation number stored in the encryption chip in the cartridge 1, and sends the collected remaining inhalation number to the control unit 24; due to the mutual cooperation and communication connection between the cartridge 1 and the power supply device 2, therefore, the reading unit 22 can read the remaining inhalation number in the memory 112 of the cartridge 1 in the power supply device;
The control unit 24 is configured to send a control instruction to the communication unit 27 according to the cigarette lighting trigger information; it is further configured to send a cigarette prohibition signal to the cigarette lighting unit 25 and the alarm unit 26 when the remaining inhalation number is 0; it is further configured to send a cigarette lighting signal to the cigarette lighting unit 25 when the remaining inhalation number is greater than 0;
the cigarette lighting unit 25 is configured to receive the cigarette lighting signal from the control unit 24 to control the operation of the atomizing device, and is further configured to receive the cigarette lighting prohibition signal from the control unit 24 to prohibit the operation of the atomizing device;
the alarm unit 26 is configured to receive the cigarette lighting prohibition signal from the control unit 24 and start;
the communication unit 27 is configured to send a cigarette lighting signal to the cartridge according to the control instruction; for the signal transceiver 111 in the cartridge to receive, and to cause the processor 113 to update the remaining inhalation number in the memory according to the preset first rule; that is, the unit inhalation number is added to the remaining inhalation number in the memory and updated.

In some embodiments, the memory 112 is configured to store the remaining inhalation number k, the preset maximum inhalation number m and cartridge information, the initial value k0 of the remaining inhalation number k is the maximum inhalation number m. The value of the maximum inhalation number m here is set by the manufacturer and cannot be changed once set. The maximum inhalation number m is also stored in the memory 112. In this way, the remaining inhalation number, the maximum inhalation number and cartridge information are stored in the memory. The cartridge information and the maximum inhalation number are pre-recorded in the memory 112, and once pre-recorded successfully, it cannot be changed. The value of the remaining inhalation number is convertible. That is, the old remaining inhalation number can be replaced with the new remaining inhalation number to update the remaining inhalation number.

When receiving the update signal, the processor 113 updates the remaining inhalation number in the memory 112 according to the preset first rule, and sends an interrupt signal to the signal transceiver 113 according to the second rule. Among them, the remaining inhalation number in the memory 112 is updated according to the preset first rule, specifically: the processor 113 receives the update signal sent by the signal transceiver 111, and subtracts the unit inhalation number from the remaining inhalation number kj in the memory 112, the calculated value is used as the new remaining inhalation number kj+1=kj-a, and sent to the memory 112 for update and storage. The unit number represented by a is usually 1, j is the number of the press, kj is the remaining inhalation number of cartridge 1 at the j-th press.

Then the power supply device matched with the cartridge reads the remaining inhalation number in the memory 112, and the preset second rule controls the opening and closing of the atomizing device. Specifically: when the remaining inhalation number kj in the read memory 112 of the power supply device is zero, the control unit in the power supply device controls the atomizing device to stop working through the cigarette lighting unit, and the alarm unit starts alarm; when the remaining number kj of the power supply device reading memory 112 is not zero, the control unit in the power supply device sends a cigarette lighting signal to the cartridge 1 according to the collected cigarette lighting trigger information.

Specifically, in this solution, the cigarette lighting signal and the interruption signal are the signals used for communication connection between the cartridge 1 and the power supply device 2. The forms of expression include electrical manifestations, such as high-frequency voltage and high-frequency pulses, the expression may further be a communication signal, such as the connection between the signal transceiver 111 and the signal transceiver 113 and the power supply device 2 through a communication network such as Bluetooth or wireless network. The cigarette lighting signal and the interrupt signal are expressed as electromagnetic waves with corresponding waveforms. In this solution, the update signal between the signal transceiver 111 and the processor 113 is usually a certain voltage signal.

### A second embodiment

This embodiment provides a cartridge. The encryption chip 11 includes a signal transceiver 111, a memory 112 and a processor 113. The difference between this embodiment and the first embodiment is:
The signal transceiver 111 is configured to receive the cigarette lighting signal sent by the power supply device 2 that cooperates with the cartridge where the encryption chip is located, and send an update signal to the processor 113; the memory 112 is configured to store the taken inhalation number and the preset maximum inhalation number, the maximum of the taken inhalation number is limited to the maximum inhalation number; the processor 113 is configured to receive the update signal sent by the signal transceiver, and update the inhalation number in the memory 112 according to the preset first rule.

In this example, the preset first rule is: the initial value of the taken inhalation number is set to 0, each time the update signal is received, the processor 113 adds the number of an unit inhalation number to the taken inhalation number as a new taken inhalation number, and updates the remaining inhalation number in the memory 112.

The implementation details of this embodiment that are different from the cartridge 1 in first embodiment 1 will be described in detail below. The following content is only implementation details provided for convenience of understanding.

The power supply device 2 reads the maximum inhalation number burned into the memory 112 of the encryption chip 11 of the cartridge 1, and calculates the remaining inhalation number based on the cigarette lighting signal. And when the remaining inhalation number is 0, the alarm unit 26 is controlled to alarm, and the atomizing device is controlled to close through the cigarette lighting unit 25. When the remaining inhalation number is greater than 0, the cigarette lighting unit 25 controls the operation of the atomizer, and the communication unit 27 sends a cigarette lighting signal to the cartridge.

In some embodiments, the signal transceiver 111 in the encryption chip 11 is configured to receive the cigarette lighting signal sent by the power supply device 2 that cooperates with the cartridge 1 where the encryption chip 11 is located, and send an update signal to the processor 113.

In some other embodiments, the power supply device is provided with an air flow or air pressure sensor, when the user inhalations, the air flow or air pressure sensor detects negative pressure and generates a inhalation signal. On the one hand, the atomizing device is controlled to start atomizing the smoke liquid to produce smoke; on the other hand, a cigarette lighting activation signal is sent to the encryption chip of the cartridge, and the encryption chip subtracts 1 from the remaining inhalation number.

### A third embodiment

This embodiment provides a cartridge 1. The structure of the cartridge 1 is substantially the same as that of the first embodiment. The cartridge 1 cooperates with the power supply device 2 of the aerosol generating apparatus, and establishes a communication connection with the power supply device 2, the cartridge 1 includes an encryption chip 11, the encryption chip 11 is configured to limit the number of cigarette lighting times of the power supply device 2 matched with the cartridge 1. Among them, the mutual cooperation between the cartridge 1 and the power supply device 2 of the aerosol generating apparatus means that the two form a physical connection, and optionally, a detachable connection.

The structure of the cartridge 1 is different from that of the first embodiment in that the encryption chip 11 includes a signal transceiver 111, a memory 112, and a processor 113, but does not include a signal transceiver. The memory 112 is configured to store the taken inhalation number and the preset maximum inhalation number, and the preset maximum inhalation number is set by the user and is changeable. Specifically, the user can make modifications through the interactive interface or control buttons of the power supply device. In some other implementations, when the power supply device is connected to a terminal or server, the preset maximum inhalation number can be modified through the terminal or server.

In some embodiments, the encryption chip does not actively detect the number of cigarette lighting times of the power supply device. Instead, when the power supply device lights a cigarette, the power supply device will actively send a cigarette lighting signal to the encryption chip. The signal transceiver 111 of the encryption chip receives the cigarette lighting signal and sends it to the processor 113, the processor 113 controls the memory 112 to increase the taken inhalation number by one.

In some embodiments, the power supply device can read the data stored in the memory 112 in the encryption chip 11, as well as the taken inhalation number and the preset maximum inhalation number, and subtract the taken inhalation number from the preset maximum inhalation number to get the remaining inhalation number. When the remaining inhalation number is equal to or less than a certain value, the alarm unit of the power supply device will issue a prompt to remind the user that the maximum inhalation number is about to be reached or has been reached. The reminder methods include but are not limited to vibration, sound, pattern, and text reminder.

### A fourth embodiment

This embodiment provides an aerosol generating apparatus, including a power supply device 2 and the cartridge 1 as described in Embodiment 1. The structure of the cartridge 1 is substantially the same as that of the first embodiment. The cartridge 1 cooperates with the power supply device 2 of the aerosol generating apparatus, and establishes a communication connection with the power supply device 2, the cartridge 1 includes an encryption chip 11, the encryption chip 11 is configured to limit the number of cigarette lighting times of the power supply device 2 matched with the cartridge 1. Among them, the mutual cooperation between the cartridge 1 and the power supply device 2 of the aerosol generating apparatus means that the two form a physical connection, and optionally, a detachable connection.

The fourth embodiment of the present disclosure provides an aerosol generating apparatus, which includes a cartridge 1 and a power supply device 2.

In some embodiments, as shown in FIG. 2, the cartridge 1 includes an encryption chip 11. The encryption chip 11 includes: a signal transceiver 111, a memory 112, a processor 113 and a signal transceiver 113; the signal transceiver 111 is configured to receive the cigarette lighting signal sent by the power supply device 2 that cooperates with the cartridge 1 where the encryption chip 11 is located, and send an update signal to the processor 113; the memory 112 is configured to store the remaining inhalation number and a preset maximum inhalation number, and the initial value of the remaining inhalation number is the maximum inhalation number; the processor 113 is configured to receive the update signal sent by the signal transceiver 111, update the remaining inhalation number in the memory 112 according to the preset first rule, and send an interrupt signal to the signal transceiver 113 according to the second rule; the signal transceiver 113 is configured to receive the interrupt signal sent by the processor 113 and send a cigarette lighting prohibition signal to the power supply device 2; the cigarette lighting prohibition signal is configured to control the cigarette lighting unit 25 of the power supply device 2 to stop working through the power supply device 2.

In some embodiments, the cartridge 1 is the cartridge in the second embodiment.

The power supply device 2 includes a reading unit 22, a collection unit 21, a control unit 24, a communication unit 27 and an alarm unit 26; the collection unit 21 is configured to collect cigarette lighting trigger information and send the information to the control unit 24; the control unit 24 is configured to send a control instruction to the communication unit 27 according to the cigarette lighting trigger information; the communication unit 27 is configured to send a cigarette lighting signal to the cartridge 1 according to the control instruction, and is further configured to send a cigarette lighting prohibition signal to the control unit 24 according to the cigarette lighting prohibition signal; in some embodiments, it is further configured to send acquisition signal instructions to the cartridge, the collection signal instruction is configured to collect the remaining inhalation number of the cartridge and send the remaining inhalation number to the control unit. In some embodiments, the collection signal instruction is configured to collect the number of smoked inhalations and the preset maximum inhalation number of the cartridge, and send the inhalation number and the preset maximum inhalation number to the control unit; the control unit 24 is configured to send cigarette lighting a control instruction to the cigarette lighting unit, and is further configured to detect the value of the remaining inhalation number, and sent a cigarette lighting prohibition signal to the cigarette lighting unit when the remaining inhalation number is 0; in some embodiments, it is further configured to control the activation of the alarm unit 26.

The reading unit 22 is configured to read the cartridge information in the encryption chip 11 of the cartridge 1 that is in contact with the power supply device 2; the storage unit 23 is configured to store cartridge information of the standard cartridge 1 that matches the power supply device 2 where it is located; the control unit 24 is configured to control the normal operation of the power supply device 2 according to whether the cartridge information sent by the reading unit 22 is consistent with the cartridge information stored in the storage unit 23.

In some embodiments, the control unit 24 is further configured to obtain the taken inhalation number by subtracting the remaining inhalation number from the maximum inhalation number preset in the encryption chip.

It is not difficult to find that this embodiment is a device embodiment corresponding to the first embodiment, and this embodiment can be implemented in cooperation with the first embodiment. The relevant technical details mentioned in the first embodiment are still valid in this embodiment, and will not be described again in order to reduce duplication. Correspondingly, the relevant technical details mentioned in this embodiment can further be applied to the first embodiment. It is worth mentioning that each unit involved in this implementation is a logic unit. In practical applications, a logical unit can be a physical unit, or a part of a physical unit, or it can be implemented as a combination of multiple physical units. In addition, in order to highlight the innovative part of the present disclosure, units that are not closely related to solving the technical problems raised by the present disclosure are not introduced in this embodiment, but this does not mean that other units do not exist in this embodiment.

### A fifth embodiment

This embodiment can obtain the taken inhalation number according to both the second embodiment and the third embodiment. In some embodiments, the power supply device is provided with a timing unit and a storage unit. The inhalation number in a period of time can be obtained based on the taken inhalation number and the timing of the timing unit. That is, the storage unit stores historical suction data, such as a day and/or a week and/or a month and/or a year.

This embodiment provides a control method for an aerosol generation system, including:
S1, the control unit 24 of the power supply device calculating the change parameter x related to the inhalation number p in the N+1th period of time and the inhalation number m in the Nth period of time, wherein the Nth period of time and the N+1th period of time are equal in length.
S2, several threshold ranges of the changing parameter x pre-stored in the terminal, and each threshold range provided with corresponding display content, the terminal displaying content corresponding to the qth threshold range to the user when receives the change parameter x and calculates that the change parameter x falls within the q-th preset threshold range.

In one of the embodiments, when the suction is m mouth during the Nth period of time, and when the suction is p mouth during the N+1th period of time, (m - p)/m =x, among them, N, m, q are natural numbers, x is 0 or a positive rational number, if satisfied,
when x<=-0.5, the first display content is output on the terminal/server, such as a crying expression;
when x>-0.5 and <=0, the second display content, such as an angry expression, is output on the terminal/server;
when x>0 and <=0.5, the second display content, such as a normal expression, is output on the terminal;
when x>0.5, the fourth display content is output on the terminal, such as a laughing expression;

In one of the embodiments, when the suction is m mouth during the Nth period of time, and when the suction is p mouth during the N+1th period of time, p / m =x, among them, N, m, q are natural numbers, x is 0 or a positive rational number, if satisfied,
when x<=0.5, the first display content, such as a laughing expression, is output to the terminal;
when x>0.5 and x<=0.85, the second display content, such as tiny expressions, is output on the terminal;
when x>0.85 and <=1.1, the third display content is output on the terminal, such as a normal (not crying or laughing) expression;
when x>1.1 and <=1.5, the fourth display content, such as a slightly angry expression, is output on the terminal;
when x> 1.5, the fifth display content is output on the terminal, such as a crying expression;

Specific scenarios as examples are described below. Take 3 days as an example.

The storage unit stores 3 days of suction data, taking the expressions of crying, slightly angry, normal, smiling, and laughing as an example.

Assume that you take 100 inhalations on the first day, and use the inhalation number on the first day as a reference on the second day.
If you take 180 inhalations the next day, change the parameter x=1.8, because when x>=1.5, a crying expression will be output on the terminal;
If you take 140 inhalations the next day and change the parameter x=1.4, because x>=1.1 and x<=1.5, a slightly angry expression will be output on the terminal;
If you take 90 inhalations the next day and change the parameter x=0.9, because x>0.85 and x<1.1, a normal expression will be output on the terminal;
If you take 40 inhalations the next day, change the parameter x=0.4, because when x <=0.5, a laughing expression will be output on the terminal;
On the third day, the inhalation number on the second day is used as the reference inhalation number. Assume that 150 inhalations were taken on the second day, then,
If you take 300 inhalations on the third day, change the parameter x=2 (300/150=2), because when x>1.5, a crying expression will be output on the terminal;
If the inhalation number is different on the third day, other expressions will be output and will not be described again here.

The above examples are only for reference and are not limited thereto. When different content is displayed to the user through the terminal according to changing parameters, the content includes but is not limited to auditory, tactile, visual content such as sound, vibration, pattern, and graphics.

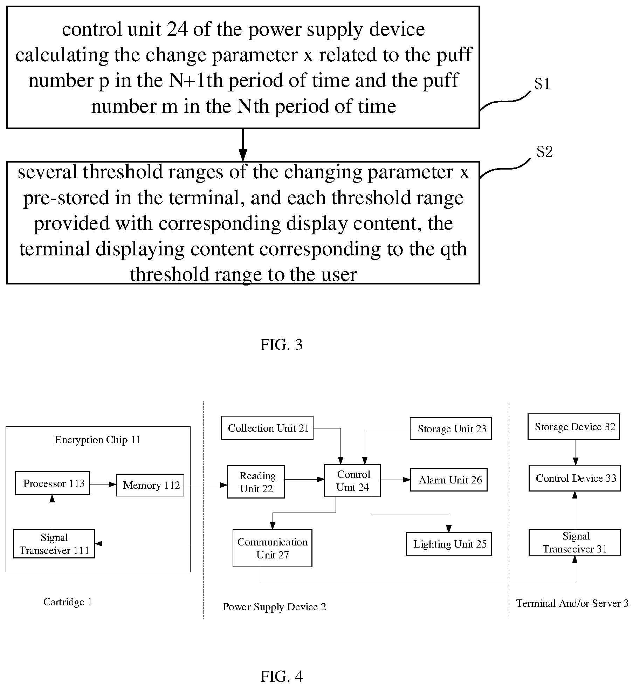

This embodiment provides an aerosol generation device system, as shown in FIG. 4, including any aerosol generation device in the aforementioned embodiments, and further includes a terminal and/or server, where N, m, and q are natural numbers, and x is 0 Or positive rational numbers.

The control unit 24 of the power supply device is further configured to calculate the change parameter x related to the inhalation number p in the N+1th period of time and the inhalation number m in the Nth period of time, and further configured to send the change parameter x to the communication unit 27, the Nth period of time and the N+1th period of time are equal in length;

### The communication unit 27 of the power supply device is further configured to send the change parameter x to the terminal and/or server

The terminal and/or server includes:
a signal transceiver 31 configured to receive the changing parameter x and send it to the control device;
a storage device 32, the memory pre-stores several threshold ranges of the change parameter x, and each threshold range is provided with corresponding display content,
a control device 33, configured to calculate that the change parameter x falls within the q-th preset threshold range, and display the content corresponding to the q-th threshold range to the user.

The above are only embodiments of the present disclosure, and common knowledge such as the known specific structures and characteristics of the solutions are not described in detail here. A person of ordinary skill in the relevant field knows all the common technical knowledge in the technical field to which the disclosure belongs before the filing date or priority date, is able to understand all the existing technologies in the field, and has the ability to apply conventional experimental methods before that date. Persons of ordinary skill in the art can, under the enlightenment given by this application, combining their own abilities to perfect and implement this solution, some typical known structures or methods should not become an obstacle for those of ordinary skill in the art to implement this application. It should be noted that those skilled in the art can make several modifications and improvements without departing from the structure of the present disclosure. These should further be regarded as the protection scope of the present disclosure, and they will not affect the effect of the present disclosure and the practicality of the patent. The scope of protection claimed in this application shall be based on the content of the claims, and the specific implementation modes and other records in the description may be configured to interpret the content of the claims.

## Claims

1. A cartridge, wherein the cartridge cooperates with a power supply device of an aerosol generating apparatus, and is electrically connected to the power supply device, the cartridge comprises an encryption chip, the encryption chip is configured to limit the number of cigarette lighting times of the power supply device cooperated with the cartridge.

2. The cartridge according to claim **1,** wherein the encryption chip comprises a signal transceiver, a memory and a processor;
the signal transceiver is configured to receive a cigarette lighting signal sent by the power supply device cooperated with the cartridge where the encryption chip is located, and send an update signal to the processor;
the memory is configured to store a remaining inhalation number, wherein an initial value of the remaining inhalation number is a preset maximum inhalation number;
the processor is configured to receive the update signal sent by the signal transceiver, and update the remaining inhalation number in the memory according to a preset first rule.

3. The cartridge according to claim **2,** wherein the processor updating the remaining inhalation number in the memory according to the preset first rule comprises:
the processor receiving the update signal sent by the signal transceiver, and subtracting the number of an unit inhalation from the remaining inhalation number in the memory, to obtain a new remaining inhalation number, and sent the new remaining inhalation number to the memory for update storage.

4. The cartridge according to claim **1,** wherein the encryption chip comprises a signal transceiver, a memory and a processor;
the signal transceiver is configured to receive a cigarette lighting signal sent by the power supply device cooperated with the cartridge where the encryption chip is located, and send an update signal to the processor;
the memory is configured to store a taken inhalation number and a preset maximum inhalation number;
the processor is configured to receive the update signal sent by the signal transceiver and update the taken inhalation number in the memory according to a preset first rule.

5. The cartridge according to claims **3** or **4,** wherein the maximum inhalation number is pre-recorded in the memory.

6. An aerosol generating apparatus, comprising the cartridge according to any one of claims **2, 3** and **5,** and the power supply device cooperated with the cartridge, wherein the power supply device comprises:
a collection unit, configured to collect a cigarette lighting trigger information and send the cigarette lighting trigger information to a control unit;
a reading unit, configured to read the remaining inhalation number stored in the encryption chip, and send the remaining inhalation number to the control unit;
the control unit, configured to send a control instruction to a communication unit and send the cigarette lighting signal to a cigarette lighting unit according to the cigarette lighting trigger information, and, detect the value of the remaining inhalation number, send a cigarette lighting prohibition signal sent to the cigarette lighting unit when the remaining inhalation number is 0;
the communication unit, configured to send the cigarette lighting signal to the cartridge according to the control instruction;
the cigarette lighting unit, configured to receive the cigarette lighting signal from the control unit to control the operation of an atomizing device, and further configured to receive the cigarette lighting prohibition signal from the control unit to prohibit the operation of the atomizing device.

7. An aerosol generating apparatus, comprising the cartridge according to claim **4,** and the power supply device cooperated with the cartridge the cartridge, and the power supply device comprises:
a reading unit, configured to read the inhalation number stored in the encryption chip, and send the collected inhalation number to a control unit;
the control unit, configured to send a control instruction to a communication unit and send the cigarette lighting signal to a cigarette lighting unit according to a cigarette lighting trigger information, and subtract the taken inhalation number from the preset maximum inhalation number to obtain the remaining inhalation number, and send a cigarette lighting prohibition signal to the cigarette lighting unit when the remaining inhalation number is 0;
the communication unit, configured to send the cigarette lighting signal to the cartridge according to the control instruction;
the cigarette lighting unit, configured to receive the cigarette lighting signal from the control unit to control the operation of an atomizing device, and receive a cigarette lighting prohibition signal from the control unit to prohibit the operation of the atomizing device.

8. An aerosol generating apparatus according to any one of claims **6** and **7,** wherein the power supply device further comprises an alarm unit;
the control unit is further configured to send the cigarette lighting prohibition signal to the alarm unit when the remaining inhalation number is 0;
the alarm unit is activated according to the cigarette lighting prohibition signal.

9. The aerosol generating apparatus according to any one of claims **6** to **8,** wherein the encryption chip of the cartridge further stores a cartridge information; the power supply device comprises:
a reading unit, configured to read a cartridge information in the encryption chip of the cartridge that is in contact with the power supply device where the reading unit is located;
a storage unit, configured to store a cartridge information of standard cartridges that match the power supply device;
a control unit, configured to control the normal operation of the power supply device according to whether the cartridge information sent by the reading unit is consistent with the cartridge information stored in the storage unit.

10. An aerosol generating apparatus system, comprising the aerosol generating apparatus according to any one of claims **7** to **10,** and further comprising a terminal and/or server,
wherein the control unit of the power supply device is further configured to calculate a change parameter x related to the inhalation number p in the N+1th period of time and the inhalation number m in the Nth period of time, and send the change parameter x to the communication unit, the Nth period of time and the N+1th period of time are equal in length, wherein N and m are natural numbers, and x is 0 or a positive rational number;
the communication unit of the power supply device, is further configured to send the change parameter x to the terminal and/or server;
the terminal and/or server comprises:
a signal transceiver device, configured to receive the changing parameter x and send it to a control device;
a storage device, configured to pre-store several threshold ranges of the change parameter x,
wherein each threshold range is provided with corresponding display content;
the control device, configured to calculate that the change parameter x falls within the q-th preset threshold range, and display the content corresponding to the q-th threshold range to an user, wherein q is a natural number.

11. The aerosol generating apparatus system according to claim **10,** wherein the change parameter x=(m-p)/p, or x=m/p.
